# FASCICULE DE BREVET EUROPEEN

(11) **EP 1 140 222 B1**
(45) Date de publication et mention de la délivrance du brevet: **03.03.2004**
(21) Numéro de dépôt: 99958319.8
(22) Date de dépôt: 14.12.1999
(51) Int. Cl.: A61L 9/01

(54) **ASSAINISSEMENT ET DESODORISATION DE L'AIR PAR BRUMISATION**
REINIGUNG UND DEODORISATION VON LUFT DURCH ZERSTÄUBUNG
SANITATION AND DEODORIZATION OF AIR BY FOG TREATMENT

(30) Priorité: 15.12.1998 FR 9815844
(43) Date de publication de la demande: 10.10.2001
(73) Titulaire: SAS GENUOL, 81000 Albi (FR)
(72) Inventeur: SAS GENUOL, 81000 Albi (FR)
(74) Mandataire: Haicour, Philippe
(86) Numéro de dépôt international: PCT/FR1999/003127
(87) Numéro de publication internationale: WO 2000/035498

(56) Documents cités:
- EP-A- 0 781 562
- FR-A- 2 706 773

## Description

### DOMAINE TECHNIQUE DE L'INVENTION

La présente invention a trait aux procédés et aux compositions pour le conditionnement, le traitement, l'assainissement et la désodorisation de l'air. Elle participe des procédés par pulvérisation ou brumisation occasionnelles, semi permanentes ou permanentes de compositions aqueuses, qui combinent le contrôle de l'hygrométrie de l'atmosphère, la diffusion de substances désodorisantes, de parfums, voire de composés désinfectants, insecticides, acaricides, etc.

### ART ANTERIEUR

Il est connu, pour lutter contre les mauvaises odeurs, de mettre en oeuvre les dérivés undécyléniques, c'est-à-dire outre l'acide undécylénique lui-même

CH₂=CH-(CH₂)₈-COOH

ses sels, ses esters, ses dérivés d'oxyéthylation, composés dont la caractéristique est de posséder une double liaison en bout de chaîne. A cette structure sont associées diverses propriétés intéressantes, dont un pouvoir antifongique et un pouvoir de neutralisation de certaines odeurs.

Les modes de désodorisation par dérivés undécyléniques de l'art antérieur sont essentiellement appliqués aux matériaux eux-mêmes qui émettent les mauvaises odeurs. C'est ainsi que par exemple, on a traité les boues des stations d'épuration (FR-A-2655856), les lisiers de porcs (FR-A-2655857, FR-A-2706773) ou encore les effluents de papeterie (FR-A-2694197), les papiers et cartons (FR-A-2742663) par introduction au sein de ces milieux de compositions undécyléniques, essentiellement l'undécylénate de méthyle ou les dérivés d'oxyéthylation de l'acide undécylénique.

On a cherché à associer les dérivés undécyléniques à des composés parfumants, et cela a donné lieu à diverses formules pour pulvérisateurs manuels qui sont soit des compositions non hydrosolubles en système solvant, soit des émulsions diluées, généralement d'undécylénate de méthyle et parfums d'ambiance, essentiellement utilisés dans les pulvérisateurs domestiques grâce auxquels on les disperse dans l'atmosphère en gouttelettes relativement grossières.

Il était très souhaitable de pouvoir disposer de solutions homogènes brumisables, c'est-à-dire diffusibles dans les atmosphères ambiantes sous forme de gouttelettes de tailles inférieures à 10 µm, dans lesquelles on pourrait associer les propriétés antiodeurs d'un dérivé undécylénique et certains composés huileux doués d'odeurs agréables et si possible de certaines propriétés utiles en assainissement, à savoir propriétés insecticides, acaricides, etc.

### EXPOSE DE L'INVENTION

On vient de trouver qu'il était possible de constituer des compositions aqueuses concentrées de ce type, si on les base sur des solutions riches, voire très riches en un sel d'acide undécylénique. Les compositions selon l'invention comportent :
- pour 100 parties d'eau,
- de 0,1 à 100 parties d'au moins un sel de l'acide undécylénique,
- de 0,5 à 50 parties d'au moins un composant huileux.
(Dans toute la suite de l'exposé, on parlera de parties en poids)

Au sens de la présente invention, on entend par composés huileux, les huiles essentielles concrètes ou non, les parfums et arômes sous forme d'huiles ou d'essences (y compris les corps synthétiques tels qu'aldéhydes aromatiques, cétones, esters, alcools) et les oléorésines. Parmi les huiles essentielles, on cite, de façon non limitative, les huiles de thym, d'eucalyptus, d'orange, de lavande, de cannelle, de pin, huiles dont les vertus stimulantes, toniques, apéritives, antistress, calmantes, relaxantes, anxiolytiques, expectorantes, en particulier stimulant l'appétit, réduisant la sensibilité au stress et aux agressions microbiennes des hommes et des animaux ou encore ayant une action bactéricide sont connues de longue date et fort appréciées.

Au sens de la présente invention, on entend par sel de l'acide undécylénique, un sel ou un mélange de sels du groupe constitué par les undécylénates de sodium, potassium, lithium, magnésium, calcium. On préfère l'undécylénate de sodium. Ces sels confèrent aux compositions selon l'invention leurs vertus propres d'agents déodorants, en même temps qu'ils jouent le rôle de solubilisants et de potentialisateurs des composés huileux qui est illustré dans les exemples fournis plus loin.

On accorde un intérêt tout particulier aux compositions qui comportent :
- pour 100 parties d'eau,
- de 20 à 100 parties d'au moins un sel de l'acide undécylénique,
- de 5 à 50 parties d'au moins un composant huileux.

Les compositions selon l'invention réalisent un équilibre tout à fait remarquable entre une aptitude à la. destruction des odeurs, dont une appréciation peut se faire à travers la mesure de l'abattement de l'ammoniac (représentatif des odeurs aminées) et de l'hydrogène sulfuré (représentatif des odeurs mercaptans) dans les atmosphères traitées, et à celle du masquage neutre ou agréable des mauvaises odeurs industrielles, celles des bâtiments d'élevage ou des locaux domestiques, dont l'efficacité peut être vérifiée au moyen d'enquêtes olfactives. Il est déjà étonnant qu'on arrive à. associer deux propriétés aussi antagonistes que la destruction d'odeurs et le masquage au sein d'une même formulation. Il est encore plus étonnant que les compositions de l'invention se révèlent des masquants nettement synergiques et donc très efficaces, comparés aux huiles entrant dans leur composition.

Ces compositions sont des liquides homogènes, limpides ou à peine opalescents. Elles sont diluables en toute proportion dans l'eau, sans démixage. Les concentrés et leurs dilutions sont parfaitement brumisables, et ne provoquent pas d'encrassement des buses de brumisation.

Les compositions de l'invention peuvent contenir une certaine quantité, de 1 à 90 parties, d'un solvant polaire hydrosoluble, pris dans le groupe des alcools et polyols hydrosolubles, notamment les polyéthylènes glycols, ainsi que leurs éthers hydrosolubles, qui participe à leur homogénéité et à leur stabilité. On préfère l'éthanol, le n-propanol, l'isopropanol, le monopropylène glycol, le dipropylèneglycol.

On peut également leur associer un ester undécylénique, remplaçant pour partie le composant huileux, qui renforce notablement le pouvoir déodorant du sel undécylénique et le pouvoir masquant / odorant du composant huileux. L'ester undécylénique selon l'invention est pris dans le groupe constitué par les esters méthylique, éthylique, propyliques et butyliques, seuls ou en mélange ; l'ester méthylique est généralement préféré, ne serait-ce que pour des raisons d'accessibilité industrielle.

Les dilutions aqueuses à 0,01 à 1 % des compositions selon l'invention sont les moyens préférés de traitement des atmosphères, bien qu'on puisse tout aussi bien brumiser les concentrés. Ces dilutions sont également des objets de l'invention. Selon l'invention, on brumise les concentrés ou leurs dilutions, de façon permanente et continue ou encore de façon intermittente et séquentielle, en les poussant sous des pressions d'au moins 80 bars, à travers des buses de brumisation d'ouverture de 10 µm ou moins. Le rythme et la durée des séquences de brumisation dépendent de la concentration en odeur ainsi que du volume d'air à traiter. On fait bénéficier les locaux ainsi traités, à la fois d'une hygrométrie confortable, d'une destruction des mauvaises odeurs, et des propriétés spécifiques de leurs composants huileux.

L'invention trouve ses champs d'application dans de très nombreuses industries. Par exemple, traitements de brumisation :
- dans les stations d'épuration, au niveau des collectes des eaux et égouts et à celui des épaississeurs et déshydratants de boues;
- dans la papeterie;
- dans l'agro-alimentaire, à l'endroit des émissions des centres d'équarrissage, des distilleries et sucreries, des centres de production de nourriture animale, manipulation de tourteaux;
- dans l'industrie chimique inorganique, au niveau des sites d'engrais, de fabrication d'acide nitrique, de chaux;
- dans l'industrie chimique organique, la production de plastiques, caoutchoucs, savons et détergents, textiles;
- dans l'élevage;
   toutes industries émettrices de molécules malodorantes fort diverses, sulfhydriques, sulfureuses, mercaptans, polysulfures, ammoniac et amines, scatole, indoles, acides organiques volatils, etc.,
- et également pour l'élimination des odeurs domestiques, sanitaires et cuisines.

On peut également utiliser les concentrés de l'invention ou leurs dilutions aqueuses pour le traitement ou l'entretien des surfaces dures, tant par pulvérisation que par étalement du fait du pouvoir détergent notable que leur communique le sel undécylénique, une propriété avantageuse supplémentaire qui n'apparaît banale qu'après coup.

### EXEMPLES

Dans les exemples 1, 2 et 3, l'appréciation de la performance de la composition résulte des résultats moyens obtenus par un jury olfactif exposé sur un site expérimental, sous le vent de la zone de retombée d'odeurs ; il donne une note d'impression globale et non différentielle, partant de "-" : extrêmement désagréable de l'atmosphère initiale. Le nombre de croix doit refléter la croissance de là satisfaction olfactive. "+++" signifie : pas d'odeur perçue. Au-delà, chaque croix correspond à l'estimation de 25% d'activité supplémentaire associant une meilleure efficacité sur plus d'odeurs différentes et aussi une meilleur efficacité surplus de concentration.

L'huile utilisée est un mélange 50/50 d'huile de thym et d'huile d'eucalyptus.

### Exemple 1

Pour illustrer la contribution spécifique du sel undécylénique, on compare une formulation selon l'invention à base avec huile et undécylénate de sodium à une formulation dans laquelle le rôle solubilisant est rempli par un tensioactif anionique industriellement très utilisé, le lauryléther sulfate de sodium, le rapprochement étant très significatif pour l'homme du métier puisqu'il s'agit de deux amphiphiles ayant à peu près même longueur de chaîne hydrocarbonée.

| N° d'essai | Solubilisant | Parties en poids pour 100 d'eau | | Note |
|---|---|---|---|---|
| | | Solubilisant | Huile | |
| 1-1 | Lauryléther-sulfate de sodium | 30 | 5 | - |
| 1-2 | Undécylénate de sodium | 30 | 5 | ++ |
| 1-3 | Undécylénate de sodium | 20 | 10 | +++ |

Les résultats 1-2 et 1-3 traduisent l'équilibre déodorant/masquant des formules selon l'invention. 1-3 détruit peut-être moins, mais masque mieux, sans défaillance dans l'hydrosolubilisation de l'huile. Le témoin manifeste aucune synergie déodorante / masquante et est très sensible à la dilution.

### Exemple 2

Formule avec undécylénate de sodium (ci-dessous C11=Na) et undécylénate de méthyle (ci-dessous C11⁼Mé) tenant lieu d'huile masquante.

| N° d'essai | Parties en poids pour 100 d'eau | | Note |
|---|---|---|---|
| | C11⁼Na | C11⁼Mé | |
| 2-1 | 30 | 5 | +++ |
| 2-2 | 20 | 10 | ++++ |

L'undécylénate de méthyle apporte à ces compositions un remarquable effet masquant, cependant sans aucune note aromatique définie qu'apprécierait le panel olfactif. Ce qui leur fait préférer des formulations de type de celles de l'exemple 3 ci-après.

### Exemple 3

Formules avec undécylénate de sodium, huile essentielle et undécylénate de méthyle.

| N° d'essai | Parties en poids pour 100 d'eau | | | Note |
|---|---|---|---|---|
| | C11⁼Na | C11⁼Mé | Huile | |
| 3-1 | 30 | 5 | 5 | +++++ |
| 3-2 | 20 | 10 | 10 | +++++++ |

Dans les formules de ce type (eau / sel undécylénique /ester undécylénique / huile aromatiques), tous les constituants entrent en synergie et contribuent d'une part à une parfaite solubilisation des composés oléosolubles, et d'autre part à un accroissement du pouvoir masquant des huiles et de l'ester et une exaltation des notes aromatiques des huiles : un résultat global fort apprécié du panel olfactif.

### Exemple 4 : concentré pour solutions désodorisantes et bactéricides.

On réalise la composition suivante, dont les ingrédients sont dosés en parties en poids :

| | |
|---|---|
| Eau | 100 parties |
| Undécylénate de sodium | 60 parties |
| Undécylénate de méthyle | 2 parties |
| Huile essentielle d'eucalyptus | 5 parties |
| Huile essentielle de thym | 5 parties |
| Ethanol | 15 parties |

La composition se présente comme une solution limpide, diluable en toutes proportions à l'eau sans démixtion.

### Exemple 5 : assainissement de l'air dans une porcherie.

La porcherie comprend une salle de 170 porcs de 70 kg de moyenne de poids vif, dont l'atmosphère a un taux moyen en NH₃ mesuré par chromatographie en phase gazeuse de 35 ppm. L'installation de traitement est un ensemble de brumisation à 6 buses alimentées sous 100 bars et émettant un flux de gouttelettes de taille inférieure à 10 µ, à raison de 15 litres par heure chacune d'une solution traitante à 1/1000 du concentré de l'exemple 1.

Après 24 heures de fonctionnement continu, la teneur de l'atmosphère en NH₃ est ramenée à 9 ppm.

### Exemple 6 : Assainissement de l'air en sortie de cheminée d'une distillerie.

Il s'agit des émissions d'une cheminée d'évacuation de la cheminée évacuant les fumées de la brûlerie de moûts de raisins à raison de 22 000 m³/h. Le taux d'ammoniac dans les fumées est de 72 ppm.

Le traitement selon l'invention consiste en une brumisation sous 100 bars, par quatre buses d'ouverture <10 µ, chacune d'un débit de 26,4 litres par heure. Solution traitante constituée d'une dilution à 3/1000 du concentré de l'exemple 1. Après 24 heures de traitement continu, le taux de NH₃ s'établit à la valeur de 9 ppm.

## Revendications

1. Composition concentrée aqueuse pour solutions à brumiser dans une atmosphère à désodoriser ou à assainir, comportant (parties en poids) :
pour 100 parties d'eau,
de 0,1 à 100 parties d'au moins un sel d'acide undécylénique,
de 0,5 à 50 parties d'au moins d'un composant huileux,
le composant huileux étant pris dans le groupe comprenant les huiles essentielles concrètes ou non, les parfums et arômes sous forme d'huiles ou d'essences et les oléorésines,
le sel de l'acide undécylénique, étant pris dans le groupe constitué par les undécylénates de sodium, potassium, lithium, magnésium, calcium.

2. Composition selon la revendication 1, comportant :
- pour 100 parties d'eau,
- de 20 à 100 parties d'au moins un sel de l'acide undécylénique,
- de 5 à 50 parties d'au moins un composant huileux.

3. Composition selon les revendications 1 ou 2, dans laquelle le sel de l'acide undécylénique est l'undécylénate de sodium.

4. Composition selon les revendications 1, 2 ou 3, comportant un ester undécylénique remplaçant pour partie le composant huileux, ledit ester étant pris dans le groupe constitué des esters méthylique, éthylique, propyliques et butyliques.

5. Composition selon la revendication 4, dans laquelle l'ester undécylénique est l'undécylénate de méthyle.

6. Composition selon l'une ou l'autre des revendications 1 à 5, comportant en outre de 1 à 90 parties d'un solvant polaire hydrosoluble, pris dans le groupe des alcools et polyols à nombre de carbones inférieur ou égal à 7 on leurs éthers.

7. Composition selon la revendication 6 dans laquelle le solvant polaire est l'éthanol, le n-propanol, l'isopropanol, le monopropylèneglycol ou le dipropylèneglycol.

8. Composition pulvérisable pour le traitement de l'air, constituée par une solution aqueuse de 0,01 à 1 % d'une composition selon l'une ou l'autre des revendications 1 à 7 dans l'eau.

9. Procédé pour le traitement de l'air, qui consiste à brumiser dans l'air une composition selon l'une ou l'autre des revendications 1 à 8.

## Claims

1. Aqueous concentrated composition for sprayable solutions to be used in fog treatment of an air to be sanitized or deodorizated, containing (by weight) :
for 100 parts of water,
from 0,1 to 100 parts of at least a salt of undecylenic acid
from 0,5 to 50 parts of at least an oily component,
the oily component being selected in the group comprising pasty or not essential oils, perfumes and oily or spirit-like aromas and oleoresins,
the undecylenic salt being selected in the group of sodium, potassium, lithium, magnesium and calcium undecylenates.

2. Composition according to claim 1, containing :
for 100 parts of water,
from 20 to 100 parts of at least a salt of undecylenic acid,
from 5 to 50 parts of at least an oily component.

3. Composition according to claims 1 or 2, where the undecylenic salt is sodium undecylenate.

4. Composition according to claims 1, 2 or 3, containing an undecylenic ester replacing partly the oily component, said ester being selected in the group of methylic, ethylic, propylic and butylic esters.

5. Composition according to claim 4, where the undecylenic ester is the methyl undecylester.

6. Composition according to either one of the claims 1 to 5, moreover containing from 1 to 90 parts of a polar water soluble solvent selected in the group of alcohols and polyols with at the utmost 7 carbon atoms and their esters.

7. Composition according to claim 6 where the polar solvent is ethanol, n-propanol, isopropanol, monoprolylen-glycol or dipropylen-glycol.

8. Sprayable composition for air treatment made of a aqueous solution of 0,01 to 1% of a composition according to either one of the claims 1 to 7.

9. Process for air treatment, consisting in spraying into air of a composition according to either one of the claims 1 to 8.

## Patentansprüche

1. Konzentrierte wässerige Zusammensetzung zur Reinigung und Deodorisation von Luft mit zerstäubenden Lösungen, welche für 100 Teilen Wasser,
von 0,1 bis 100 Teilen mindenstens eines Salzes von Undecylensäure,
von 0,5 bis 50 Teilen mindenstens einer öligen Komponente, (Gew. Teilen), umfasst,
wobei die ölige Komponente unter den Gruppen die die dickflüssigen oder nicht Duftöle, die Düftstoffe und öligen oder essenzartigen Aromen und die Harzöle umfassen gewählt ist,
wobei das undecylenische Salz in der Gruppe von Natrium-, Kalium-, Lithium-, Magnesium-, Kalziumundecylenaten gewählt ist.

2. Zusammensetzung nach Anspruch 1, umfassend für 100 Teilen Wasser,
von 20 bis 100 Teilen mindenstens eines Salzes von Undecylensäure;
von 5 bis 50 Teilen mindenstens einer öligen Komponente.

3. Zusammensetzung nach Ansprüchen 1 oder 2, wobei das Salz von Undecylensäure Natriumundecylenat ist.

4. Zusammensetzung nach Ansprüchen 1, 2, oder 3 umfassend einen Undecylenester der die ölige Komponente teilweise ersetzt, wobei der Ester in der Gruppe der Methyl-, Äthyl-, Propyl- und Butylester gewählt ist.

5. Zusammensetzung nach Anspruch 4, wobei der Undecylenester der Methylundecylenester ist.

6. Zusammensetzung nach einer der Ansprüchen 1 bis 5, welche weiter von 1 bis 90 Teilen eines polaren Lösungsmittels enhält, der in der Gruppe der Alkoholen und Polyalkoholen mit höchstens 7 Kohlenstoffatomen und ihrer Ätheren gewählt ist.

7. Zusammensetzung nach Anspruch 6, wobei das polare Lösungsmittel Äthanol, n-Propanol, Isopropanol, Propylenglykol oder Dipropylenglykol ist.

8. Zerstäubbare Zusammensetzung zur Reinigung von Luft bestehtend aus einer wässerigen Lösung von 0,01 bis 1°% einer Zusammensetzung nach eine oder andere der Ansprüchen 1 bis 7.

9. Verfahren zur Reinigung von Luft der in Zerstäubung in der Luft mit einer Zusammensetzung nach einer oder anderer der Ansprüchen 1 bis 8 besteht.
